# EUROPEAN PATENT APPLICATION

(11) **EP 4 260 879 A1**
(43) Date of publication of application: **18.10.2023**
(21) Application number: 21921375.8
(22) Date of filing: 28.12.2021
(51) Int. Cl.: A61L 2/10, A61L 9/20, B32B 27/00, B32B 27/18, B32B 27/30, D06N 7/04, E04F 13/02, E04F 13/07

(54) **PROTECTION PROCESSING METHOD, INACTIVATION FUNCTION INSTALLATION METHOD, SHEET MATERIAL, AND STRUCTURE**

(30) Priority: 22.01.2021 JP 2021008397
(71) Applicant: Ushio Denki Kabushiki Kaisha, Tokyo 100-8150 (JP)
(72) Inventor: SUZUKI, Takayuki, Tokyo 100-8150 (JP)
(74) Representative: Tomerius, Isabel
(86) International application number: PCT/JP2021/048814
(87) International publication number: WO 2022/158274

(57) **Abstract**

Provided is a method of protection treatment capable of undergoing inactivation treatment using ultraviolet light in a target space where a sheet material whose main component is a chlorine-based resin has been disposed or is planned to be disposed, while suppressing the progress of discoloration and deterioration of the sheet material. The target space is a space where a light source emitting ultraviolet light having light output in a specific wavelength band belonging to within a range from 190 nm to 235 nm has already been disposed or is planned to be disposed. The method of protection treatment includes the steps of attaching a base material whose main component is a chlorine-based resin, to an interior surface of the target space or a surface of an object disposed in the target space, and forming a protective layer on a surface of the base material, the protective layer containing a material that exhibits resistance to the ultraviolet light.

## Description

### TECHNICAL FIELD

The present invention relates to a method of protection treatment for a target space in which ultraviolet light is used for the inactivation treatment of bacteria and/or viruses. The present invention also relates to a method of providing an inactivation function for a target space, the method encompassing the protection treatment. In addition, the present invention relates to a sheet material suitable for achieving the method of protection treatment. Furthermore, the present invention relates to a structure in which the protection treatment and the inactivation treatment and the inactivation function have been provided.

### BACKGROUND ART

Microorganisms (bacteria, fungi, etc.) and viruses present in a space or on the surface of an object can cause infectious diseases in humans and non-human animals, and there is a concern that the spread of infectious diseases may threaten people's lives. In particular, infectious diseases are more likely to spread in facilities where people frequently gather, such as medical facilities, schools, and government offices, or vehicles, such as automobiles, trains, buses, airplanes, and ships, thereby necessitating an effective method of inactivating bacteria and/or viruses (hereinafter collectively referred to as "pathogens").

Conventionally, ultraviolet light irradiation has been known as a method of inactivating pathogens. DNA exhibits the highest absorption characteristic near a wavelength of 260 nm. A low-pressure mercury lamp has a high emission spectrum near a wavelength of 254 nm. Hence, a technology of sterilizing using a low-pressure mercury lamp has been widely adopted.

However, ultraviolet light with this wavelength band is known to adversely affect human bodies when irradiated to humans. The skin is divided into three parts starting from the part near the front surface: epidermis, dermis, and deeper subcutaneous tissue. The epidermis is further divided into four layers starting from the part near the surface: stratum corneum, granular layer, spinous layer, and basal layer. When radiated to human bodies, ultraviolet light having a wavelength of 254 nm penetrates the stratum corneum and reaches the granular layer and the spinous layer, and possibly the basal layer, where it is absorbed by the DNA of cells present in these layers. This results in the risk of skin cancer. Hence, it is difficult to actively use ultraviolet light having the wavelength band in places where people can be present.

Patent Document 1 below discloses that ultraviolet light having a wavelength of 240 nm or longer (UVC light) is harmful to human bodies, and that ultraviolet light having a wavelength of less than 240 nm has a limited degree of adverse effects on human bodies compared to ultraviolet light having a wavelength of 240 nm or longer. In addition, the results of irradiation experiments at wavelengths of 207 nm and 222 nm are specifically described.

### CITATION LIST

Patent Document 1: Japanese Patent No. 6025756

### SUMMARY OF THE INVENTION

### Technical Problem

The inventor has diligently studied the inactivation treatment of bacteria and/or viruses using ultraviolet light having a wavelength of less than 240 nm, more specifically, ultraviolet light having a light output in a specific wavelength band belonging to within a range from 190 nm to 235 nm, in a space where humans may be present, such as rooms in buildings, vehicles, and corridors (hereinafter referred to as "target spaces"). As a result, the inventor newly discovered the following issues.

Building sheets (sheet materials) such as wallpaper and flooring are typically disposed on the inner walls, ceilings, and floor surfaces of rooms and corridors in buildings. Also, in vehicles such as automobiles and airplanes, it is common that some kinds of protective sheet materials are disposed on the interior parts. Furthermore, front surfaces of items mounted in rooms, corridors, and vehicles (e.g., desks, chairs, various devices, etc.) may also be provided with protective sheet materials.

Previously, effects on the sheet materials have not been examined when the sheet materials are irradiated with ultraviolet light having a wavelength of less than 240 nm, more specifically, ultraviolet light having a light output in a specific wavelength band belonging to within a range from 190 nm to 235 nm. The present inventor has diligently examined this point and found that when the sheet materials composed of chlorine-based resins represented by polyvinyl chloride (PVC) are irradiated with the ultraviolet light even at an illuminance level as low as 1 mW/cm² or less, the rate of discoloration and deterioration of the sheet materials is fast. Since PVC is a material commonly used for building sheets such as wallpaper and flooring, the irradiation of the ultraviolet light for inactivating pathogens and viruses in rooms in buildings, etc., may cause discoloration to the building sheets over time.

Although building sheets are expected to discolor and deteriorate over time even through normal use, the rate of discoloration and deterioration increases when exposed to the ultraviolet light. As a result, this may discourage facility managers and owners from introducing inactivation treatment to target spaces using the ultraviolet light.

In addition, since PVC is a material commonly used as a film for front surface protection, it may also be assumed to be used for applications other than building sheets. Hence, there is a concern that the rate of deterioration and discoloration may increase with respect to the front surface of items mounted in the target space.

In view of the above problems, it is an object of the present invention to provide a method of protection treatment capable of undergoing inactivation treatment using ultraviolet light in a target space where a sheet material whose main component is a chlorine-based resin has been disposed or is to be disposed, while suppressing the progress of discoloration and deterioration of the sheet material. It is another object of the present invention to provide a method of providing an inactivation function for a target space where the protection treatment that suppresses the progress of discoloration or deterioration of the sheet material has been included.

It is yet another object of the present invention to provide a sheet material that suppresses the progress of discoloration and deterioration even when the sheet material is disposed in a target space where inactivation treatment using ultraviolet light is performed. It is yet another object of the present invention to provide a structure that suppresses the progress of discoloration and deterioration on the front surface even when the structure is disposed in a target space where inactivation treatment using ultraviolet light is performed.

### Solution to the Problem

In the present invention, there is provided a method of protection treatment according to the present invention in a target space where inactivation of bacteria and/or viruses is performed, the target space being a space where a light source emitting ultraviolet light having light output in a specific wavelength band belonging to within a range from 190 nm to 235 nm has already been disposed or is planned to be disposed, the method including the steps of:
attaching a base material whose main component is a chlorine-based resin, to an interior surface of the target space or a surface of an object disposed in the target space; and
forming a protective layer on a surface of the base material, the protective layer containing a material that exhibits resistance to the ultraviolet light.

In the present specification, the term "inactivation" refers to a concept that encompasses eliminating bacteria and/or viruses or causing them to lose their infectivity or toxicity, and the term "pathogens" refers to microorganisms such as bacteria and fungi (mold). Hereinafter, "bacteria and/or viruses" may also be collectively referred to as "pathogens".

In the present specification, the term "main component" is used as a term to represent the component with the maximum content on a mass basis. The term "main component" typically refers to a component having 40 mass percent or more, more typically a component having 50 mass percent or more, and particularly typically a component having 60 mass percent or more.

The above-mentioned Patent Document 1 describes that ultraviolet light having a wavelength band shorter than 240 nm is used to suppress the degree of adverse effects on human bodies. The target space where the method of protection treatment according to the present invention is applied is a space where inactivation treatment of bacteria and/or viruses is planned to be performed while suppressing the adverse effects on human bodies by the irradiation of ultraviolet light having light output in a specific wavelength band belonging to within a range from 190 to 235 nm.

The ultraviolet light is preferably such that, in the emission spectrum, the ratio of the integral value of the light output from 240 nm to 300 nm to the integral value of the light output from 190 nm to 235 nm is less than 1%. The use of such ultraviolet light allows the inactivation treatment of bacteria and/or viruses while suppressing the adverse effects on human bodies. As a light source emitting such ultraviolet light, excimer lamps filled with luminescent gases such as KrCl, KrBr, and ArF, or solid-state light sources such as LED and laser diodes can be employed. In this case, the peak wavelength of the ultraviolet light may be located in a range from 190 nm to 235 nm.

The ultraviolet light having light output in a wavelength band from 200 to 230 nm is more preferable. This enables a high inactivation effect to be achieved while ensuring more safety for human bodies. The ultraviolet light having this wavelength range is believed to have few adverse effect on human bodies, even if irradiated for a relatively long period of time.

Examples of chlorine-based resins include polyvinyl chloride, polyvinylidene chloride, chlorinated polyethylene, and chlorinated polypropylene. All of these have C-CI bonds.

When a chlorine-based resin is irradiated with ultraviolet light having light output in a specific wavelength band belonging to within a range from 190 nm to 235 nm, C-Cl bonds in the resin may be disconnected by the light energy the ultraviolet light possesses. The amount to which and the rate at which this C-Cl bond is disconnected depends on the illuminance and irradiation time (i.e., amount of irradiation dose) of the ultraviolet light. Hence, for example, when a target space where wallpaper made of a chlorine-based resin is disposed on the interior surface is irradiated with the ultraviolet light, discoloration and deterioration of the wallpaper are accelerated compared to the case in which there is no irradiation of ultraviolet light.

In contrast, according to the above-mentioned method of protection treatment, a base material whose main component is a chlorine-based resin is mounted on the interior surface of the target space or the surface of an object disposed in the target space, and a protective layer including a material exhibiting resistance to the ultraviolet light is formed on the surface of the base material. Thus, even when the interior surface of the target space or the surface of an object disposed in the target space is irradiated with the ultraviolet light, the presence of the protective layer formed on the surface of the base material attached to the interior surface or the surface of the object prevents the ultraviolet light from penetrating the base material. This suppresses the progress of discoloration and deterioration of the base material.

The interior surface may include an inner wall surface, a floor surface, and a ceiling surface of the target space.

In the present invention, there is provided a method of protection treatment for a target space where inactivation of bacteria and/or viruses is performed, the target space being a space where a light source emitting ultraviolet light having light output in a specific wavelength band belonging to within a range from 190 nm to 235 nm has already been disposed or is planned to be disposed, the method including the steps of:
preparing a specified material including a base material whose main component is a chlorine-based resin and a protective layer including a material that exhibits resistance to the ultraviolet light, the protective layer being formed on a surface of the base material; and
attaching the specified material to an interior surface of the target space or a surface of an object disposed in the target space.

According to the above-mentioned method, formed is a specific material including a base material whose main component is a chlorine-based resin and a protective layer including a material exhibiting resistance to the ultraviolet light on the surface of the base material formed on the interior surface of the target space or the surface of an object disposed in the target space. Accordingly, as is similar to the above-described invention, even if the interior surface of the target space or the surface of an object disposed in the target space is irradiated with the ultraviolet light, the presence of the protective layer formed on the surface of the base material disposed on the interior surface or the surface of the object prevents the ultraviolet light from penetrating the base material. This suppresses the progress of discoloration and deterioration of the base material.

In addition, in the present invention, there is provided a method of protection treatment for a target space where inactivation of bacteria and/or viruses is performed, the target space being a space where a light source emitting ultraviolet light having light output in a specific wavelength band belonging to within a range from 190 nm to 235 nm has already been disposed or is planned to be disposed, the method including the step of applying a protective layer containing a material that exhibits resistance to the ultraviolet light to a surface of a base material whose main component is a chlorine-based resin attached to an interior surface of the target space or a surface of an object disposed in the target space.

According to the above-mentioned method, even if a base material whose main component is a chlorine-based resin, such as wallpaper or sheet materials for surface protection, has already been attached in the target space, a protective layer can be applied to the upper surface of the base material. Accordingly as is similar to the above-described invention, even if the interior surface of the target space or to the surface of an object disposed in the target space is irradiated with the ultraviolet light, the presence of the protective layer formed on the surface of the base material disposed on the interior surface or the surface of the object prevents the ultraviolet light from penetrating the base material. This suppresses the progress of discoloration and deterioration of the base material.

This method can be implemented, for example, by dissolving the material exhibiting resistance to the ultraviolet light in a predetermined solvent and then spraying this solvent onto the surface of the base material with a spray or the like.

The protective layer may be preferably made of a fluorine-based resin as its main component. Any fluorine-based resin is suitable, and examples of fluorine-based resins include polychlorotrifluoroethylene (PCTFE) and polytetrafluoroethylene (PTFE). The present inventor's diligent research has confirmed that coating with a fluorine-based resin is more effective in suppressing the progress of discoloration and deterioration than that with glass coating and titanium coating. In addition, a fluorine-based resin has no effect on the color or decoration of the base material (wallpaper, flooring, etc.) that exists in the lower layer because of its high transparency to visible light.

It is more preferable that the protective layer may contain an ultraviolet light absorbent in a fluorine-based resin.

Some fluorine-based resins exhibit properties of transmitting a certain proportion of the ultraviolet light. In such a case, even if a protective layer whose main component is a fluorine-based resin is formed on the surface of the base material, part of the ultraviolet light may penetrate the protective layer and is radiated to the base material, which is located underneath the protective layer, posing a risk of accelerating the rate of discoloration and deterioration compared with those in the normal use.

In contrast, since the above configuration has the protective layer made of a fluorine-based resin containing an ultraviolet light absorbent, ultraviolet light traveling through the fluorine-based resin is absorbed by the ultraviolet light absorbent contained in the resin, resulting in a significant reduction in the amount of ultraviolet light irradiation to the base material. This significantly reduces the progress of discoloration and degradation of the base material.

As ultraviolet light absorbents, for example, benzophenone-based materials, benzotriazole-based materials, salicylic acid ester-based materials, acrylonitrile derivative-based, and other materials that exhibit ultraviolet light absorption properties are suitably used. These may be used alone or in a combination of two or more types. The ultraviolet light absorbent preferably has a transmittance of less than 30% to ultraviolet light having light output in the specific wavelength band belonging to within a range from 190 nm to 235 nm, more preferably less than 20%, and particularly preferably less than 10%.

It is preferable that 0.1 to 10 parts by mass of the ultraviolet light absorbents are added to 100 parts by mass of fluoropolymer materials, and it is particularly preferable that 0.5 to 5 parts by mass of the ultraviolet light absorbents are added thereto.

The light source may be used to inactivate bacteria and/or viruses in the target space by irradiating the base material with the ultraviolet light at an illuminance of 1 mW/cm² or less.

Even when the ultraviolet light is radiated at such a low illuminance as described above, inactivation of bacteria and/or viruses present in the target space can still be performed.

Meanwhile, as mentioned above, it is known that ultraviolet light radiated to humans has adverse effects on human bodies. As of the filing date of this application, the American Conference of Governmental Industrial Hygienists (ACGIH) and JIS Z 8812 (Methods for Measuring Hazardous Ultraviolet Light Radiation), and other standards, specify the threshold limit value (TLV) for each wavelength with respect to the amount of ultraviolet light irradiation per day (8 hours) to human bodies. In other words, when ultraviolet light is used in an environment where humans are present, it is recommended that the illuminance and irradiation time of ultraviolet light is determined such that the cumulative amount of irradiation of ultraviolet light radiated within a given time is within the TLV standard value.

In the configuration in which ultraviolet light is radiated at a low illuminance as described above, it is easy to perform inactivation while keeping the cumulative amount of irradiation within the TLV standard value, even during the period when humans are present.

And even in the case where ultraviolet light is radiated at such a low illuminance, problems such as discoloration and degradation of a chlorine-based resin may become apparent as the irradiation time becomes longer and the cumulative amount of irradiation increases. In contrast, the method described above suppresses the rate of discoloration and degradation of the base material because the protective layer is formed on the surface of the base material whose main component is a chlorine-based resin, even if the cumulative amount of irradiation increases due to long-term irradiation with low-illuminance ultraviolet light over a long period of several months to several years. The term "irradiation over a long period of several months to several years" does not necessarily mean that the irradiation over a 24-hour duration, which constitutes a day, constantly continues for several months or several years. It may mean that the aspect including a period with ultraviolet light irradiation and a period with no ultraviolet light irradiation continues for several months to several years. Another example is that one or more days with no ultraviolet light irradiation may be included in several months or several years.

It is preferable that the protective layer may have a transmittance of 50% or more to visible light in a wavelength band from 400 nm to 600 nm. The high transparency to visible light has no effect on the color or decoration of the base material (wallpaper, flooring, etc.) present underneath the protective layer. In addition, it is more preferable that the protective layer has a transmittance of 60% or more to visible light, and especially preferable 75% or more.

The present invention includes a method of providing an inactivation function for providing an inactivation function of bacteria and/or viruses in a target space, the method including the steps of:
disposing a light source emitting ultraviolet light having light output in a specific wavelength band belonging to within a range from 190 nm to 235 nm in the target space; and
performing the method of protection treatment described above.

The method described above provides a function capable of inactivating bacteria and/or viruses in a target space in which a base material whose main component is a chlorine-based resin is disposed, while inhibiting the progress of discoloration and deterioration of the base material.

In addition, the present invention includes a sheet material for protection that is mounted in a target space in which the inactivation of bacteria and/or viruses is performed, the sheet material including: a base material whose main component is a chlorine-based resin; and a protective layer that is formed on a surface of the base material and that contains a material exhibiting resistance to ultraviolet light having light output in a specific wavelength band belonging to within a range from 190 nm to 235 nm.

The sheet material suppresses the progress of discoloration and deterioration due to the irradiation with the ultraviolet light, even if used as a protective sheet for interior surfaces in a target space where a light source emitting the ultraviolet light is disposed for the inactivation treatment of bacteria and/or viruses, or for the surface of an object disposed in the target space.

The present invention also includes a structure including an inactivation function for bacteria and/or viruses, the structure including:
a light source emitting ultraviolet light having a light output in a specific wavelength band belonging to within a range from 190 nm to 235 nm; and
a protective layer that contains a material exhibiting resistance to the ultraviolet light and that is disposed within an area possibly irradiated with the ultraviolet light; and
a base material whose main component is a chlorine-based resin, and is disposed underneath the protective layer.

Examples of the structure include spaces in which humans may be present, such as rooms in buildings, vehicles, and corridors.

### Effects of the Invention

The present invention enables the inactivation treatment of bacteria and/or viruses in a target space while suppressing the progress of discoloration and deterioration of a sheet material (base material), even though the base material whose main component is a chlorine-based resin is used in the target space.

### BRIEF DESCRIPTION OF DRAWINGS

FIG. 1 is a schematic diagram illustrating an example of a scene in which the inactivation treatment of bacteria and/or viruses in accordance with the present invention is expected to be performed.
FIG. 2 is a schematic drawing illustrating a structure of an excimer lamp as an example of a light source.
FIG. 3 is a graph illustrating an example of the spectrum of ultraviolet light emitted from the light source.
FIG. 4 is a schematic view illustrating a state in which ultraviolet light is radiated from the light source onto wallpaper.
FIG. 5 is a graph illustrating the transmission spectrum of PTFE (polytetrafluoroethylene).
FIG. 6A is a photograph of each sample before irradiation with ultraviolet light.
FIG. 6B a photograph of each sample after irradiation with ultraviolet light.
FIG. 7A is a graph of verification results to describe that low-illuminance ultraviolet light serves to inactivate bacteria.
FIG. 7B is a graph of verification results to describe that low-illuminance ultraviolet light serves to inactivate viruses.

### DESCRIPTION OF EMBODIMENTS

The embodiments of the method of protection treatment, the method of providing an inactivation function, a sheet material, and a structure according to the present invention will be described with reference to the drawings as appropriate. In the following, "inactivation treatment of bacteria and/or viruses" may simply be abbreviated as "inactivation treatment".

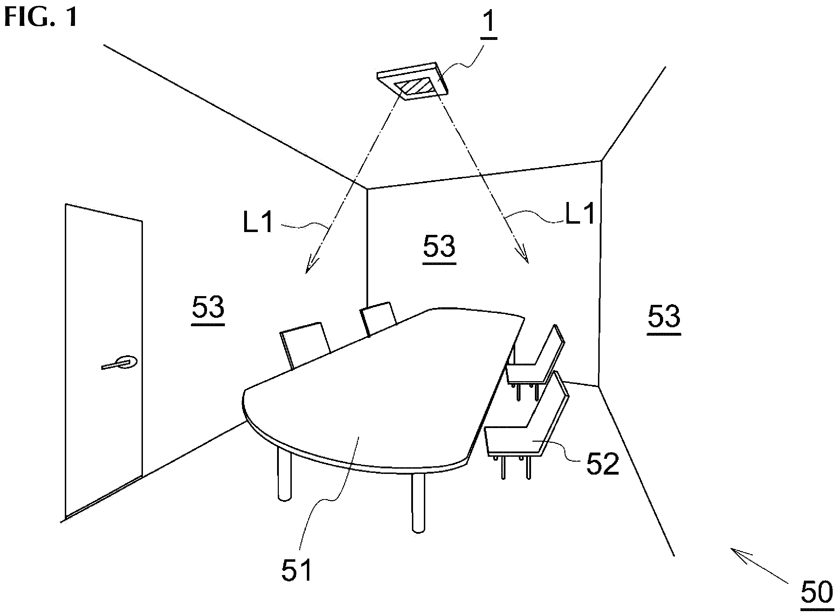

FIG. 1 is a schematic view illustrating an example of a scene in which the inactivation treatment according to the present invention is expected to be performed. FIG. 1 illustrates a situation in which a light source 1 emitting ultraviolet light L1 is disposed in a room 50, such as a conference room. The light source 1 is disposed to perform inactivation treatment of pathogens in the room 50. In other words, the room 50 corresponds to a "target space" in the example shown in FIG. 1. More precisely, the light source 1 is disposed to perform inactivation treatment on a desk 51, chairs 52, wallpaper 53 that are present in the room 50, and a space itself of the room 50.

FIG. 1 also corresponds to a diagram illustrating the room 50 in a scene in which the method of protection treatment and/or the method of providing inactivation function according to the present invention is implemented. The processes for implementing each of the above methods will be described later.

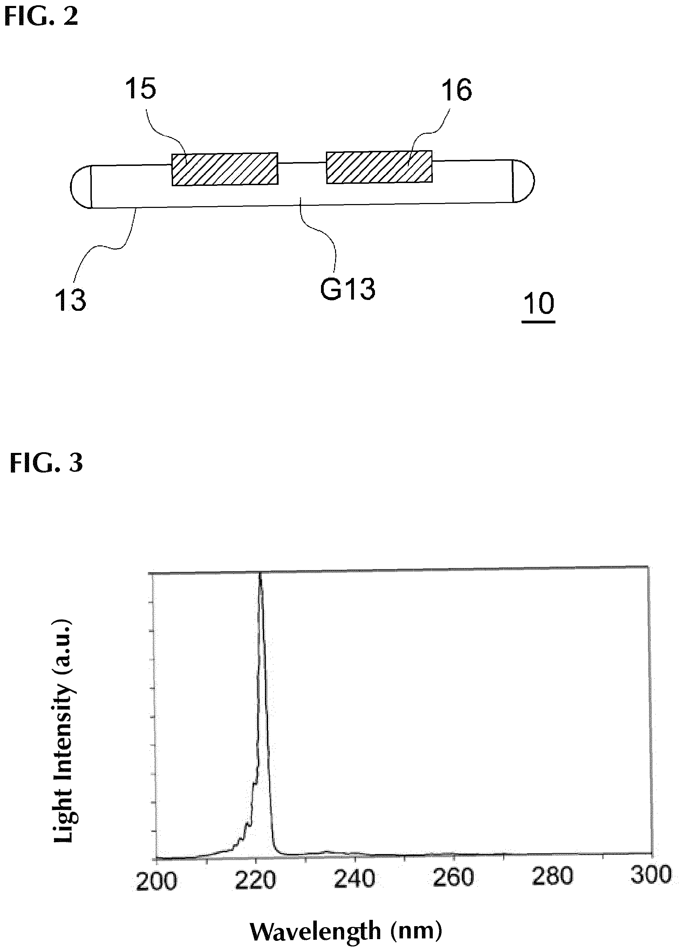

The light source 1 is configured to emit the ultraviolet light L1 having light output in a specific wavelength band belonging to within a range from 190 nm to 235 nm. The light source 1 is configured to be an excimer lamp as an example. The excimer lamp 10, as a more detailed specific example, includes a light-emitting tube 13 filled with luminescent gas G13 and a pair of electrodes (15, 16) arranged to be in contact with the outer surface of the light-emitting tube, as shown in a schematic view in FIG. 2.

The luminescent gas G13 consists of a material that emits the ultraviolet light L1 having light output in a specific wavelength band belonging to within a range from 190 nm to 235 nm during excimer emission. Examples of the luminescent gas G13 include KrCl, KrBr, and ArF

When the luminescent gas G13 contains KrCl, for example, the excimer lamp 10 emits the ultraviolet light L1 with a peak wavelength of near 222 nm. When the luminescent gas G13 contains KrBr, the excimer lamp 10 emits the ultraviolet light L1 with a peak wavelength of near 207 nm. When the luminescent gas G13 contains ArF, for example, the excimer lamp 10 emits the ultraviolet light L1 with a peak wavelength of near 193 nm. The excimer lamp 10 may be configured to emit the ultraviolet light L1 with a longer wavelength with respect to the emission wavelength of the excimer light by applying a phosphor to the tube wall of the light-emitting tube 13. FIG. 3 is a graph illustrating an example of the spectrum of the ultraviolet light L1 emitted from excimer lamp 10 in which the luminescent gas G13 contains KrCl. According to the spectrum shown in FIG. 3, the ratio of the integral value of light output from 240 nm to 300 nm to the integral value of light output from 190 nm to 235 nm is less than 1%.

However, the light source 1 can be any light source as long as it is configured to emit the ultraviolet light L1 having light output in a specific wavelength band belonging to within a range from 190 nm to 235 nm. In other words, the light source 1 may be configured to be a solid-state light source such as an LED or a laser diode instead of the excimer lamp 10. Even when the light source 1 includes the excimer lamp 10, the excimer lamp 10 can be formed of any shape other than that shown in FIG. 2.

As described above, the light source 1 performs inactivation treatment of pathogens to items in the room 50 (desk 51, chairs 52, wallpaper 53, etc.) and in the space of the room 50 by radiating the ultraviolet light L1 in the room 50. In the example of FIG. 1, the surface of the wallpaper 53 corresponds to the "interior surface of the target space", and the surfaces of the desk 51 and the chairs 52 correspond to the "surface of an object disposed in the target space".

For the inactivation treatment of pathogens, the light source 1 preferably radiates the ultraviolet light L1 at an illuminance of 1 mW/cm² or less. It will be described later that the inactivation of pathogens can be achieved even at such a low illuminance.

In particular, irradiating the room 50 with the ultraviolet light L1 at an illuminance as low as 1 mW/cm² or less enables inactivation treatment to be performed while keeping the cumulative amount of irradiation within the TLV standard value even if humans are present in the room 50.

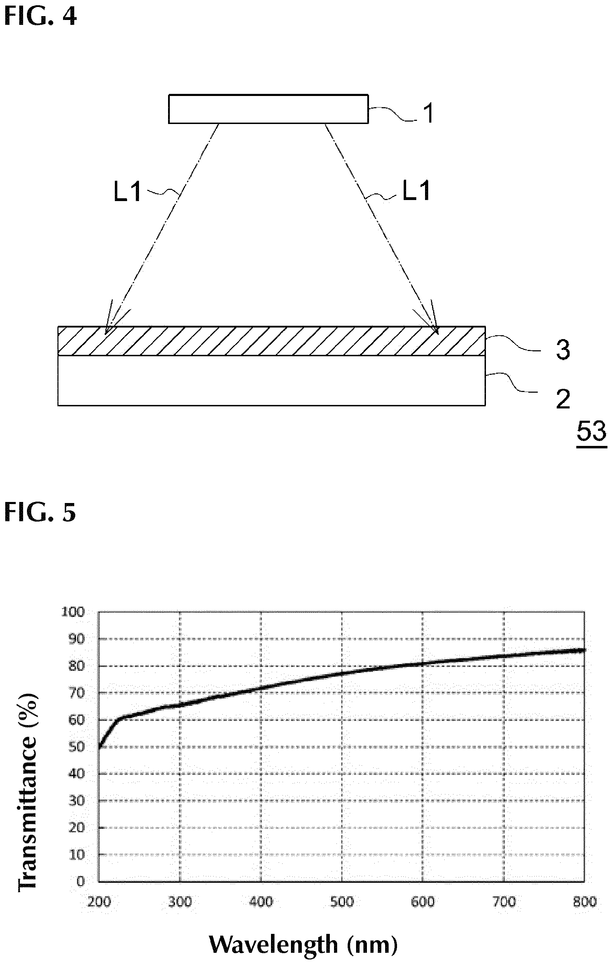

FIG. 4 is a schematic view illustrating a state in which the light source 1 irradiates the wallpaper 53 with the ultraviolet light L1. In the present invention, the wallpaper 53 includes a base material 2 and a protective layer 3. The protective layer 3 is formed on the surface of the base material 2 (more precisely, on the surface closer to the internal space of the room 50). The formation of the protective layer 3 prevents the surface of the base material 2 from being exposed to the internal space.

The base material 2 is made of chlorine-based resin as its main component. Examples of the chlorine-based resin typically used include polyvinyl chloride, polyvinylidene chloride, chlorinated polyethylene, and chlorinated polypropylene.

In the present embodiment, the protective layer 3 consists of a fluorine-based resin as the main component and contains an ultraviolet light absorbent. Typically, an ultraviolet light absorbent is dispersed in a fluorine-based resin.

Examples of fluorine-based resins that can be used include polychlorotrifluoroethylene (PCTFE), polytetrafluoroethylene (PTFE), and polyvinylidene fluoride (PVDF). In particular, a fluorine-based resin is preferably a material having a transmittance of 50% or more for visible light in the wavelength band from 400 nm to 600 nm. FIG. 5 is a graph indicating the transmission spectrum of PTFE as an example of a fluorine-based resin. The transmission spectrum shown in FIG. 5 was obtained by placing a resin made of PTFE opposite a light source, receiving light transmitted through this resin with a light receiver, and breaking down the ratio of the intensity of the received light to the intensity of light emitted from the light source by wavelength. FIG. 5 confirms that PTFE has a transmittance of 70% or more for visible light in a wavelength band from 400 nm to 600 nm.

Examples of the ultraviolet light absorbent that can be used include benzophenone-based material, benzotriazole-based material, salicylic acid ester-based material, acrylonitrile derivative-based material, and other materials that exhibit ultraviolet light absorption properties. The ultraviolet light absorbent is preferably a material that can be mixed or dispersed in a fluorine-based resin and that has low transmittance for the ultraviolet light L1 having light output in a specific wavelength band particularly belonging to within a range from 190 nm to 235 nm. This transmittance is preferably less than 30%, more preferably less than 20%, and further preferably less than 10%. The ultraviolet light absorbent is preferably a material that exhibits a transmittance of 50% or more for visible light in the wavelength band from 400 nm to 600 nm.

The protective layer 3 can be produced, for example, by kneading and processing an ultraviolet light absorbent such as a benzophenone-based material into a fluorine-based resin such as PTFE. Alternatively, as another method, the constituent materials of a fluorine-based resin and the constituent materials of an ultraviolet light absorbent can be dissolved together in a solvent such as butyl acetate, dimethyl ether, or the like. By applying this solvent to the upper surface of the base material 2 and allowing it to volatilize, the protective layer 3 is formed on the upper surface of the base material 2.

When the light source 1 radiates the ultraviolet light L1 toward the wallpaper 53, the formation of the protective layer 3 on the surface of the base material 2 restricts the progress of the ultraviolet light L1 in the protective layer 3, thereby significantly reducing the amount of ultraviolet light irradiated to the base material 2 located underneath the protective layer 3. As a result, the C-Cl bonds contained in the constituent materials of the base material 2 are less likely to be broken by the light energy derived from the ultraviolet light L1. Therefore, the progress of discoloration and degradation of the base material 2 is suppressed.

In addition, since the protective layer 3 has high transmittance to visible light, even the formation of the protective layer 3 on the surface of the base material 2 fails to affect the color or decoration of the base material 2 located underneath the protective layer 3. In other words, the progress of discoloration and deterioration caused by the irradiation of the ultraviolet light L1 is suppressed while the color and decoration inherent to the wallpaper 53 are utilized.

In the above embodiment, the case in which the wallpaper 53 is composed of the base material 2 and the protective layer 3 is described as an example. However, in the case of the example shown in FIG. 1, it is assumed that the desk 51 or the chairs 52 may also be provided with a protective material on their surfaces. In such a case, it is suitable that the protective member is configured to include the base material 2 and the protective layer 3. This suppresses, as is similar to the case of the wallpaper 53, the progress of discoloration and deterioration caused by the irradiation of the ultraviolet light L1 without affecting the color or decoration of the desk 51 or chairs 52.

The following describes the method of protection treatment and method of providing inactivation function (hereinafter referred to as the "method of the present invention"). The method of the present invention can be implemented in two aspects described below. Both aspects will be described using the room 50 shown in FIG. 1 as an example.

### First Aspect

The first aspect is mainly assumed in the case of newly building the room 50 and is performed through each of the following processes.

### Process S1

The wallpaper 53 is attached to the inner walls of the room 50 for interior use. As described above with reference to FIG. 4, this wallpaper 53 includes the protective layer 3 formed on the surface of a base material 2. When the wallpaper 53 is attached, the protective layer 3 may be provided on the upper surface of the base material 2 after the base material 2 has been attached, or the base material 2 with the protective layer 3 that has already been formed (corresponding to the "specified material") may be attached.

A piece of flooring formed with the protective layer 3 on the surface of the base material 2 may also be attached, as similar to the wallpaper 53, on the floor surface of the room 50. The above-described wallpaper 53 may also be attached to the ceiling surface of the room 50.

When the protective layer 3 is attached after the base material 2 has been attached, the protective layer 3 having a sheet-like form may be attached to the upper surface of the base material 2, or a solvent in which a fluorine-based resin and an ultraviolet light absorbent are dissolved may be applied onto the surface of the base material 2. In the former case, the adhesive used in the lamination process is preferably a material with high resistance to the ultraviolet light L1.

### Step S2

The light source 1 emitting the ultraviolet light L1 is disposed at a predetermined location in the room 50. In the case of FIG. 1, the light source 1 is disposed on the ceiling surface of the room 50. The order of process S2 and process S1 is interchangeable as needed.

The above-mentioned processes S1 and S2 provides the protection treatment on the base material 2 such as the wallpaper 53 against the ultraviolet light L1 in addition to the inactivation function of pathogens in the newly-built room 50. In the case where the light source 1 has already been disposed in the room 50 because process S2 has been executed earlier, the above-mentioned process S1 is executed to apply the protection treatment to the interior surface of the room 50 against the ultraviolet light L1.

### Second Aspect

The second aspect is mainly assumed in the case where the light source 1 for inactivation treatment is mounted in the existing room 50.

### Process S1a

The base material 2 as wallpaper is usually already disposed on the interior surface of the existing room 50. Then, the protective layer 3 is attached to the surface of the base material 2. In this case, as also described above in the first aspect, the protective layer 3 having a sheet-like form may be attached to the upper surface of the base material 2, or a solvent in which a fluorine-based resin and an ultraviolet light absorbent are dissolved may be applied onto the surface of the base material 2. However, in the case of this second aspect, the latter method is suitable in view of workability. In particular, it is suitable to spray the solvent in which a fluorine-based resin and an ultraviolet light absorbent are dissolved in a form of aerosol using compressed gas.

Even for the floor surface of the room 50, the protective layer 3 may also be attached to the surface of the existing flooring. For the ceiling surface of the room 50, the protective layer 3 may also be attached to the surface of the existing base material 2, which is an existing wallpaper, in a similar manner as described above.

### Process S2

As is similar to the first aspect, the light source 1 emitting the ultraviolet light L1 is disposed at a predetermined location in the room 50 before and after the execution of process S1. This provides a protection treatment to the base material 2 such as wallpaper in addition to an inactivation function for pathogens in the existing room 50.

Hereinafter, the results of the verification will be described.

### Verification 1

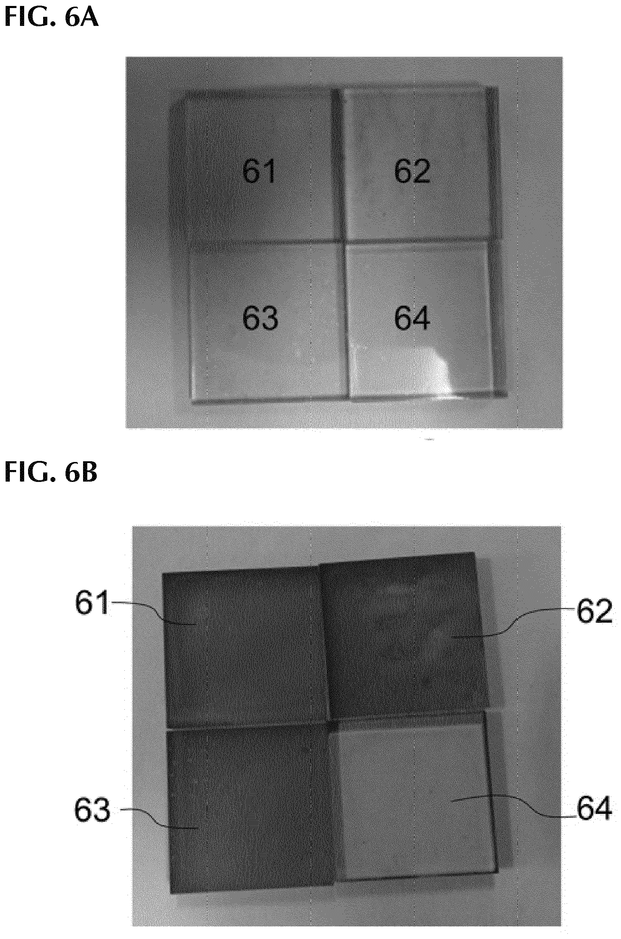

FIG. 6A is a photograph of samples 61 to 64 before the verification experiment. The sample 61 is a plate made of polyvinyl chloride (PVC) as an example of chlorine-based resin. Each of the samples 62 to 64 is subject to treatment on the surface of the sample 61. Details are as follows.

The sample 62 was subject to a glass-coating treatment on its surface. Specifically, the glass coating was prepared by spraying a volatile solvent such as xylene or ethyl benzene in which an acrylic resin and glass beads were dissolved onto its surface.

The sample 63 was subject to a titanium-coating treatment on its surface. Specifically, the sample 63 was prepared by spraying a 0.85% titanium dioxide aqueous solution onto its surface and allowing it to dry.

The sample 64 was subject to a fluorine-coating treatment on its surface. Specifically, the sample 64 was prepared by spraying a volatile solvent such as dimethyl ether in which PTFE as a fluorine-based resin is dissolved onto its surface. The samples 62 to 64 are each subject to the treatment on the surface of the sample 61; however, they all exhibit nearly the same level of visible light transparency as the sample 61 and are nearly identical in appearance.

FIG. 6B shows a photograph of the samples 61 to 64 after the continuous irradiation of the ultraviolet light L1 at a peak wavelength of 222 nm emitted from a KrCl excimer lamp at an illuminance of 0.44 mW/cm² for 644 hours. This cumulative amount of irradiation is equivalent to 127 years when converted by the daily standard value of TLV for ultraviolet light with a wavelength of 222 nm, which is 22 mJ/cm². However, it is conceivable that irradiation in the actual operation will reach this cumulative amount of irradiation in a shorter period of time than 127 years because no restriction is imposed on the ultraviolet light irradiation when humans are absent.

According to FIG. 6B, discoloration was barely observed for the fluorine-based resin-coated sample 64, whereas the untreated sample 61, the glass-coated sample 62, and the titanium-coated sample 63 all showed discoloration. As a result, it can be seen that the protective layer 3, which has a fluorine-based resin as its main component, has high resistance to the ultraviolet light L1 and suppresses discoloration and deterioration of polyvinyl chloride (PVC), which simulates the base material 2 formed underneath the protective layer 3.

The experiment shown in FIG. 6B confirmed that the sample 62 with glass-coating treatment and the sample 63 with titanium-coating treatment barely exhibited effects on the discoloration or degradation of the base material 2 (here, polyvinyl chloride) against the ultraviolet light L1. In all of these treatments, it is presumed that forming a layer with a low resistance to the ultraviolet light L1 on the surface of the base material 2 allows discoloration to progress due to an increase in the amount of the irradiation dose of the ultraviolet light L1, as is similar to the untreated sample 61. In other words, in Verification 1, discoloration was suppressed only in the sample 64 coated with a fluorine-based resin, but it is presumed that discoloration and degradation can be suppressed as is similar to the sample 64, even when a coating made of other materials exhibiting resistance to the ultraviolet light L1 is applied to the surface of the base material 2.

However, a fluorine-based resin transmits a certain amount of the ultraviolet light L1 having light output in a specific wavelength band belonging to within a range from 190 nm to 235 nm. Hence, if the protective layer 3 is composed only of a fluorine-based resin, discoloration and degradation of the base material 2 may progress as the amount of irradiation dose of the ultraviolet light L1 increases. This progress may be at a slower rate than that in the case where the protective layer 3 is absent. From this viewpoint, it is preferable that the protective layer 3 contains an ultraviolet light absorbent in addition to the fluorine-based resin.

For example, instead of the sample 64, spraying a volatile solvent such as dimethyl ether in which PTFE as a fluorine-based resin and a hydroxybenzophenone-based material as an ultraviolet light absorbent is dissolved onto the surface of the sample 61 for coating can further suppress the progress of discoloration and degradation of the base material 2 than those in the sample 64.

### Verification 2

Four types of resins such as a polycarbonate (PC) resin, an ABS resin, a polyethylene terephthalate (PET) resin, and a PVC resin, were prepared as the base material 2, and they each were irradiated with the ultraviolet light L1 with a peak wavelength of 222 nm at an illuminance of 0.04 mW/cm² to confirm discoloration of the resin.

After the irradiation was started, the PVC resin turned brownish in color, and the discoloration was clearly confirmed with naked eyes at 58.1 hours after the start of the irradiation (equivalent to approximately one year in terms of the TLV standard value per day, which is 22 mJ/cm²). In contrast, the PC resin, the ABS resin, and the PET resin did not show much difference in color visually compared to the situation before the irradiation with the ultraviolet light L1. Furthermore, the PVC resin became discolored to an even darker brownish color with the continued irradiation of the ultraviolet light L1.

A PVC resin is a substance having C-Cl bonds unlike a PC resin, an ABS resin, and PET resin. From this viewpoint, it is presumed that the irradiation of the ultraviolet light L1 causes the disconnection of C-Cl bonds, resulting in the degradation and discoloration of a PVC resin. In contrast, it is presumed that a PC resin, an ABS resin, and a PET resin are inherently more resistant to the ultraviolet light L1 than a PVC resin because they do not have C-Cl bonds.

### Verification 3

The following describes a point in which ultraviolet light is effective in the inactivation treatment of bacteria and/or viruses in the room 50 even when the ultraviolet light L1 is irradiated in the room 50 at a low illuminance of 1 mW/cm2 or less with reference to the experimental results.

In a petri dish with a diameter of 35 mm, 1 mL of Staphylococcus aureus at a concentration of approximately 10⁶ /mL is placed. The petri dish was irradiated from above with the ultraviolet light L1 with a peak wavelength of 222 nm emitted from a KrCl excimer lamp under different irradiation conditions. The solution in the petri dish after irradiation with the ultraviolet light L1 was then diluted with saline to a predetermined ratio, and 0.1 mL of the diluted solution was seeded onto standard agar medium. The seeded medium was cultured for 24 hours at 37°C and 70% humidity, and the number of colonies was counted.

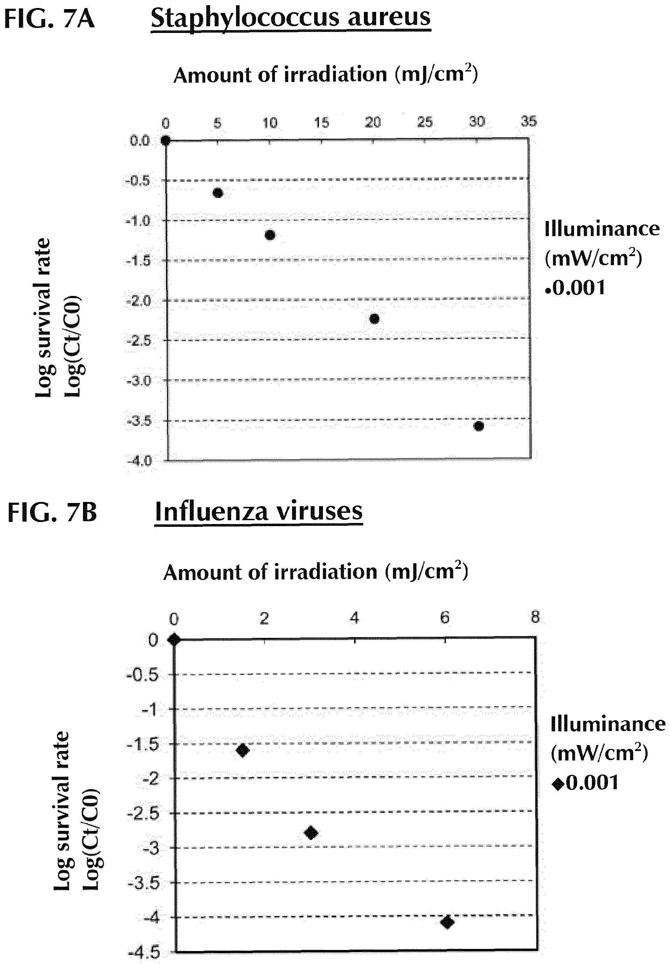

FIG. 7A is a graphical representation of the results of the above experiment, where the horizontal axis corresponds to the amount of irradiation of the ultraviolet light L1 and the vertical axis corresponds to the survival rate of Staphylococcus aureus. The vertical axis corresponds to the log value of the ratio of the number of colonies of Staphylococcus aureus after the irradiation of the ultraviolet light L1 to the number of colonies of Staphylococcus aureus at the time before the irradiation thereof.

FIG. 7A confirms that the inactivation of Staphylococcus aureus can be achieved even when the illuminance of the ultraviolet light L1 is extremely low at 0.001 mW/cm². It has also been confirmed that the ultraviolet light L1 has an inactivation effect on other pathogens such as Bacillus cereus and Bacillus subtilis.

As another verification, FIG. 7B indicates the results of the similar verification performed on influenza viruses. This result also confirmed that the ultraviolet light L1 enables the inactivation of the viruses. For example, the amount of ultraviolet light irradiation of 3 mJ/cm² can be achieved by the irradiation for 50 minutes at an illuminance of 0.001 mW/cm², and also achieved by the irradiation for 5 minutes at an illuminance of 0.01 mW/cm². FIG. 7B confirms that the ultraviolet light L1 enables the inactivation of viruses. It has also been confirmed that the ultraviolet light L1 has an inactivation effect on other viruses such as the feline coronavirus.

As described above, the ultraviolet light L1 is capable of inactivating bacteria and/or viruses in the room 50 even when the light source 1 radiates the ultraviolet light L1 at a low illuminance of 1 mW/cm² or less. The degree of the inactivation effects of bacteria and/or viruses depends on the cumulative amount of irradiation of the ultraviolet light L1.

### Reference Signs List

- 1: Light source
- 2: Substrate
- 3: Protective layer
- 10: Excimer lamp
- 13: Light-emitting tube
- 50: Room
- 51: Desk
- 52: Chair
- 53: Wallpaper
- 61: Sample
- 62: Sample
- 63: Sample
- 64: Sample
- G13: Luminescent gas
- L1: Ultraviolet light

## Claims

1. A method of protection treatment in a target space where inactivation of bacteria and/or viruses is performed, the target space being a space where a light source emitting ultraviolet light having light output in a specific wavelength band belonging to within a range from 190 nm to 235 nm has already been disposed or is planned to be disposed, the method comprising the steps of:
attaching a base material whose main component is a chlorine-based resin, to an interior surface of the target space or a surface of an object disposed in the target space; and
forming a protective layer on a surface of the base material, the protective layer containing a material that exhibits resistance to the ultraviolet light.

2. A method of protection treatment for a target space where inactivation of bacteria and/or viruses is performed, the target space being a space where a light source emitting ultraviolet light having light output in a specific wavelength band belonging to within a range from 190 nm to 235 nm has already been disposed or is planned to be disposed, the method comprising the steps of:
preparing a specified material including a base material whose main component is a chlorine-based resin and a protective layer including a material that exhibits resistance to the ultraviolet light, the protective layer being formed on a surface of the base material; and
attaching the specified material to an interior surface of the target space or a surface of an object disposed in the target space.

3. A method of protection treatment for a target space where inactivation of bacteria and/or viruses is performed, the target space being a space where a light source emitting ultraviolet light having light output in a specific wavelength band belonging to within a range from 190 nm to 235 nm has already been disposed or is planned to be disposed, the method comprising the step of applying a protective layer containing a material that exhibits resistance to the ultraviolet light to a surface of a base material whose main component is a chlorine-based resin attached to an interior surface of the target space or a surface of an object disposed in the target space.

4. The method of protection treatment according to one of claims 1 to 3, wherein the light source is used to inactivate bacteria and/or viruses in the target space by irradiating the base material with the ultraviolet light at an illuminance of 1 mW/cm² or less.

5. The method of protection treatment according to one of claims 1 to 3, wherein the protective layer is made of a fluorine-based resin as its main component.

6. The method of protection treatment according to claim 5, wherein the protective layer contains an ultraviolet light absorbent in the fluorine-based resin.

7. The method of protection treatment according to one of claims 1 to 3, wherein the protective layer has a transmittance of 50% or more to visible light in a wavelength band from 400 nm to 600 nm.

8. The method of protection treatment according to one of claims 1 to 3, wherein the interior surface includes an inner wall surface, a floor surface, and a ceiling surface of the target space.

9. A method of providing an inactivation function for providing an inactivation function of bacteria and/or viruses in a target space, the method comprising the steps of:
disposing a light source emitting ultraviolet light having light output in a specific wavelength band belonging to within a range from 190 nm to 235 nm in the target space; and
performing the method of protection treatment described in any one of claims 1 to 3.

10. A sheet material for protection that is attached in a target space in which inactivation of bacteria and/or viruses is performed, the sheet material comprising:
a base material whose main component is a chlorine-based resin; and
a protective layer that is formed on a surface of the base material and that contains a material exhibiting resistance to ultraviolet light having light output in a specific wavelength band belonging to within a range from 190 nm to 235 nm.

11. The sheet material according to claim 10, wherein the protective layer is made of a fluorine-based resin as its main component.

12. The sheet material according to claim 11, wherein the protective layer contains an ultraviolet light absorbent in the fluorine-based resin.

13. A structure providing an inactivation function for bacteria and/or viruses, the structure comprising:
a light source emitting ultraviolet light having a light output in a specific wavelength band belonging to within a range from 190 nm to 235 nm; and
a protective layer that contains a material exhibiting resistance to the ultraviolet light and that is disposed within an area possibly irradiated with the ultraviolet light; and
a base material whose main component is a chlorine-based resin, and is disposed underneath the protective layer.
